# EUROPEAN PATENT SPECIFICATION

(11) **EP 2 158 926 B1**
(45) Date of publication and mention of the grant of the patent: **22.05.2013**
(21) Application number: 09168493.6
(22) Date of filing: 24.08.2009
(51) Int. Cl.: A61M 1/00, A61M 11/02

(54) **Aspirator assembly**
Sauganordnung
Ensemble aspirateur

(30) Priority: 02.09.2008 US 231343
(43) Date of publication of application: 03.03.2010
(73) Proprietor: Sebban, Eric, 75020 Paris (FR)
(72) Inventor: Sebban, Eric, 75020 Paris (FR)
(74) Representative: Laget, Jean-Loup

(56) References cited:
- EP-A2- 1 180 373
- WO-A2-2008/058160
- US-B1- 6 328 718

## Description

### BACKGROUND

This invention relates to a hand held aspirating assembly structured to aspirate fluid from the nasal cavity of individuals specifically including, but not limited to, infants and children. A source of negative pressure is battery powered and a receiving chamber is cooperatively structured to house a removable collection container. A predetermined portion of the aspirated fluid, such as mucus, is collected in the container and collectively disposed therewith after the aspiration procedure has been completed.

The ordinary cold, as well as more serious respiratory infections, can result in the blockage of the nasal passage or cavity due to the accumulation of mucus therein. In the case of adult individuals the collected mucus can frequently or at least partially be removed by forced expulsion when the individual blows his or her nose. However, in the case of small children or infants the self induced expulsion of the mucus by blowing ones nose typically has not been learned. Accordingly, in many instances fluid, and in particular the mucus, built up in the nasal cavity or passage must be removed by other means. Removal of the mucus is also important for the general health of the individual at least to the extent of preventing or minimizing the drainage of the mucus back into the throat, lungs. In addition, inordinate collection of certain fluids in the nasal passage or cavities significantly increases the possibility of infection.

In cases involving infants and small children as well as adult individuals not capable of the self expulsion of mucus and other collections from the nasal cavity, an aspiration procedure is commonly applied. Conventionally known aspiration procedures are frequently accomplished by using a hand manipulated device such as a flexible or elastic material "squeeze" bulb connected to or otherwise directly associated with a nozzle structure or other appropriate applicator structured to be received within the nasal passage of an individual. With the interior of the squeeze bulb disposed in fluid communication with the nozzle, it is disposed to at least partially enter the nasal cavity or passage so as to positioned in communicating relation with the mucus and/or other collected fluid therein. By virtue of the elastic or flexible nature of the material from which the squeeze bulb is formed, it can be manually collapsed, by the application of squeezing force thereon, and subsequently released. Such manipulation results in an at least partial vacuum or negative pressure being on the collected fluids within the nasal cavity.

While such procedures and accompanying apparatus have been known for many years, it is commonly recognized that problems and disadvantages are associated with the application thereof. More specifically, because the negative pressure generated by the squeeze bulb is frequently insufficient, mucus and other fluids are not effectively removed from the nasal passage. Also, because of the inadequacy of the negative pressure developed, it is frequently required that the nozzle portion of the squeeze bulb be inserted repeatedly. Such a repetitive application frequently results in a "packing" or physical forcing of the collected mucus farther up into the nasal cavity or passage.

Accordingly, because of the ineffectiveness and overall inefficiency of known procedures and techniques, as generally described above, there is a need in this area of a more effective and efficient aspirator assembly. Such a proposed and improved assembly should be structured to facilitate the ability of parents, caregivers or other individuals to quickly and effectively remove collected fluid from the nasal passage in order to allow proper breathing by the afflicted individual. In an attempt to overcome problems set forth above, mechanical devices have been devised which attempt to overcome the disadvantages and problems of the manually operable squeeze bulb structure. However, while such prior attempts may have been considered at least minimally operative, many known devices still do not quickly and efficiently remove the collected fluid from the blocked nasal cavity in an effective manner.

Therefore, there still exists a need in this area for an aspirator assembly which is capable of being used on infants, small children as well as adult individuals. Such proposed assemblies should be capable of use without necessitating the discomfort of the individual being aspirated. Further, an improved and proposed aspirator assembly should be capable of being hand held, at least partially self contained and dimensioned and structured to assure proper operation and effective aspiration of the individual.

The aim of the present invention also consists in improving aspirating assembly structured intended to aspirate fluid from the nasal cavity of individuals.

### SUMMARY OF THE INVENTION

This invention as defined by the claims is directed to an aspirating assembly dimensioned and configured to be hand held and operative by a single hand of a user and functional to aspirate the nasal cavity of an individual including, but not limited to, infants and children. The assembly is self contained at least to the extent of including a battery operated vacuum pump or other negative pressure source disposed within a casing. A cover is removably attached to the casing and at least partially defines a receiving chamber into which aspirated fluid from the nasal cavity is received. In addition, a collection container is removably disposed within the receiving chamber in receiving relation to a predetermined portion of the aspirated fluid, which is at least partially defined by the heavier material mucus and other substances. For purposes of clarity, the term "aspirated fluid" is herein meant to include a lighter, gaseous phase such as, but not necessarily limited to, air which is removed from the nasal cavity during the aspirating procedure. In addition, "aspirated fluid" is also meant to include a "heavier" material phase, generically described herein as mucus. Accordingly, the aspirated mucus is accurately referred to herein as a "predetermined portion" of the aspirated fluid as described in greater detail hereinafter.

The aspirator assembly further includes an inlet assembly connected to or formed as part of the removable cover. The inlet assembly is structured to further facilitate the direction of flow of the heavier, predetermined portion of the aspirated fluid into the collection container. Thereafter, the container and collected mucus portion of the aspirated fluid are collectively disposed of after the aspiration procedure has been completed. Accordingly, the contamination and possible reduction of health hazards are accomplished by allowing for the disposal and replacement of the collection container as versus its cleaning, after use.

As will be explained in greater detail hereinafter, the negative pressure source and its power supply in the form of a battery pack are housed within a hollow interior of the casing and are respectively configured and dimensioned to facilitate the casing being held and operated by a single hand of a user or operator. As such, the vacuum or negative pressure created by the negative pressure source communicates with the receiving chamber and with the aforementioned inlet assembly. Upon the creation of a sufficient negative pressure, the direct application of the inlet assembly into an appropriate portion of the nasal cavity, will serve to remove the trapped and/or collected aspirated fluid. As set forth above and more fully explained hereinafter, the cooperative disposition and structuring of the inlet assembly in aligned relation with the interior of the collection container facilitates the collection of the heavier mucus or predetermined portion of the aspirated fluid into the interior of the container.

Concurrently as both air and mucus, collectively and at least partially defining the aspirated fluid, are drawn into the receiving chamber through the aforementioned inlet assembly, the air, or gaseous portion of the aspirated fluid, will be effectively separated from the mucus and directed at least partially away from the interior of the collection container. Moreover, the gaseous portion of the aspirated fluid will be directed out from the interior of the receiving chamber and along a predetermined "path of fluid flow". Accordingly, the aforementioned path of fluid flow is also disposed and structured to establish fluid communication between the receiving chamber and the inlet assembly and the negative pressure source. As a result, at least a partial vacuum will develop within the interior of the receiving chamber and be maintained during the operation of the vacuum pump or other negative pressure source. Further, the disposition and overall structure of the path of fluid flow is such as to facilitate passage of air and/or lighter, gaseous phase of the aspirated fluid out from the interior of the receiving chamber, while restricting passage of the predetermined, heavier portion of the aspirated fluid along the path of the fluid flow. As a result, the predetermined heavier portion of the aspirated fluid will be collected within the container for the collective disposal of the container and the collected mucus after completion of the aspiration procedure.

Other structural and operative features of the aspirating assembly include operative controls easily activated by one or more fingers of the user and a display, facilitating visualization of the various operative features and performance characteristics of the aspirating assembly during its activation, operation and overall use. Yet additional operative features and components of the aspirating assembly may include a sound generating assembly structured to produce music or other appropriate sounds, which may be especially appealing and therefore serve as a diversion to infants and children, during the aspirating procedure.

Furthermore, additional structural and operative features of the aspirating assembly include a dispensing assembly structured and disposed to selectively deliver at least one liquid, gas, or other solution to the nasal cavity, which, in at least one embodiment, may assist with the collection of aspirated fluid and/or the treatment of infections, colds, or mucus buildup. In particular, the dispensing assembly may include a removably or fixedly connected receptacle, a connecting assembly, and an outlet assembly disposed in a fluid communication with one another to facilitate the delivery of fluid from the receptacle to the nasal cavity or passage.

In particular, the object of the present invention to provide an assembly structured to aspirate a nasal cavity, said assembly comprising: a casing including an at least partially hollow interior, a cover structured to at least partially define a receiving chamber on an interior thereof when said cover is connected to said casing, a negative pressure source disposed within said casing and in fluid communication with said receiving chamber, and an inlet assembly disposed in communicating relation with said receiving chamber and structured to direct aspirated fluid into said receiving chamber, the assembly comprising a collection container removably disposed within said receiving chamber and disposed to collect the aspirated fluid therein, characterized in that said collection container comprises at least one or a plurality of channels formed in its exterior surface allowing a gaseous portion of the aspirated fluid entering said receiving chamber to be directed out from the interior of said receiving chamber through the channels while restricting the passage of a heavier portion or mucus portion of the aspirated fluid.

Favourably, the dispensing assembly comprises a receptacle and an outlet assembly, said receptacle being disposed in a fluid communication with said outlet assembly.

Advantageously, the dispensing assembly comprises a plurality of interchangeable receptacles.

In particular, the assembly further comprises a container removably disposed within said receiving chamber and disposed to collect the aspirated fluid therein.

Favourably, the receptacle and said outlet assembly are disposed in said fluid communication with one another via at least one connector assembly.

In particular, the connector assembly comprises at least one tube, said at least one tube comprising an at least partially hollow interior configuration.

Advantageously at least a portion of said tube is disposed in an external relation relative to said cover.

Preferably, the cover comprises a groove disposed on an external surface thereof, said groove being cooperatively structured to receive said portion of said tube therein.

Preferentially, the assembly further comprises a nozzle connected to said outlet assembly, said nozzle being structured and disposed to direct said at least one liquid from said outlet assembly to the nasal cavity.

In particular, said outlet assembly is operatively disposed in a substantially adjacent relation to said inlet assembly.

Preferably, said nozzle is disposable in substantially surrounding relation to at least a portion of said inlet assembly and said outlet assembly.

Advantageously said nozzle is dimensioned and configured to enter into the nasal cavity.

In particular, said dispensing assembly is structured to selectively deliver said liquid to the nasal cavity.

Preferentially, said dispensing assembly further comprises an actuator assembly structured to at least partially control a flow of said liquid to the nasal cavity.

Preferably, said actuator assembly is at least partially coupled to said connector assembly.

These and other objects, features and advantages of the present invention will become clearer when the drawings as well as the detailed description are taken into consideration.

### DESCRIPTION OF THE PREFERRED EMBODIMENT

For a fuller understanding of the nature of the present invention, reference should be had to the following detailed description taken in connection with the accompanying drawings in which:
Figure 1 is a side view of the aspirating assembly of the present invention.
Figure 2 is a front view of the embodiment of Figure 1.
Figure 3 is an interior view of the embodiment of Figures 1 and 2 representing various operative components contained within a handheld casing.
Figure 4 is a sectional view of the interior of a receiving chamber as well as cooperative components including a cover and disposable collection container.
Figure 5 is a sectional view similar to but structurally distinguishable from the embodiment of Figure 4.
Figure 6 is an exterior view of the cover represented in Figures 4 and 5.
Figure 7 is an exterior view of a nozzle assembly as represented in Figures 1-3.
Figure 8 is an exterior view of the disposable collection container as represented in Figures 4 and 5.
Figure 9 is an exterior view of yet another embodiment of the aspirator assembly disclosed in accordance with the present invention.
Figure 10 is an exploded view of the aspirator assembly illustrated in Figure 9.

Like reference numerals refer to like parts throughout the several views of the drawings.

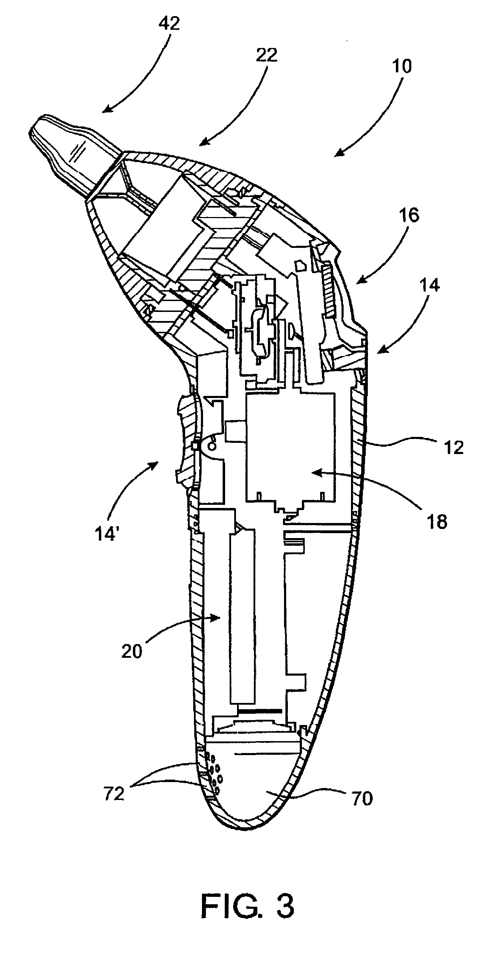

As shown in the accompanying Figures, the present invention is directed to an aspirator assembly generally indicated as 10 including an at least partially hollow interior casing 12, as represented in Figure 3. The casing 12 is dimensioned and configured to be hand held and includes a plurality of operator buttons or like control members generally indicated as 14. The controls 14 facilitate activation and operation of the aspirator assembly 10 by a single hand of an operator. In conjunction therewith, a display assembly generally indicated as 16 is disposed in a readily observable location on the casing 12 and is cooperatively structured with appropriate control and operating circuitry (not shown) to allow visualization of the operational and performance characteristics of the aspirator assembly 10. Additional activation controls 14' may also be disposed on the casing 12 and generally, but not exclusively, may be operatively connected to appropriate control circuitry to serve as an on/off or other switch for the aspirator assembly 10.

Additional operative components of the aspirator assembly 10, includes a negative pressure source generally indicated as 18 which may be include a vacuum pump or other appropriate structure. As such, the negative pressure source 18 is powered by a battery pack or battery assembly generally indicated as 20, which is also contained in a hollow interior portion of the casing 12. As set forth above, appropriate control and activating buttons 14 and 14' are electrically connected to provide control circuitry so as to activate and regulate operation of the aspirator assembly.

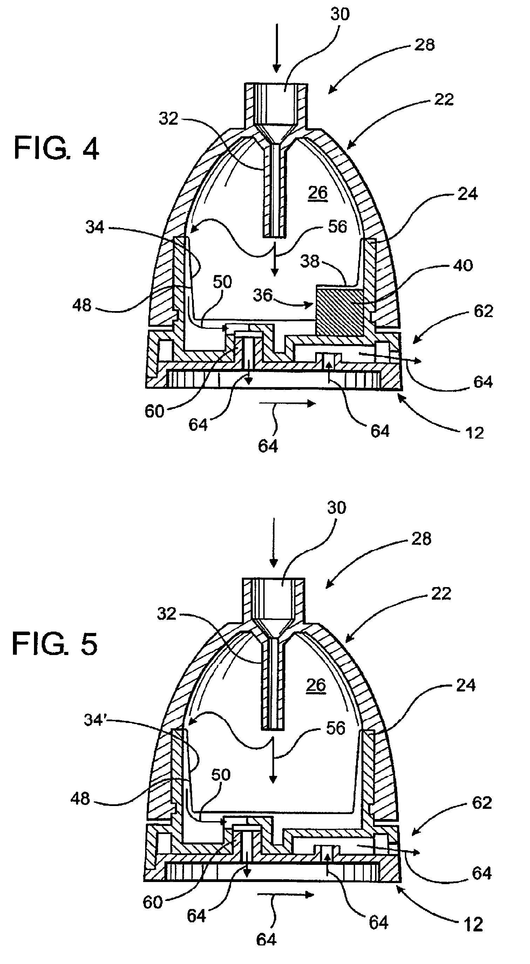

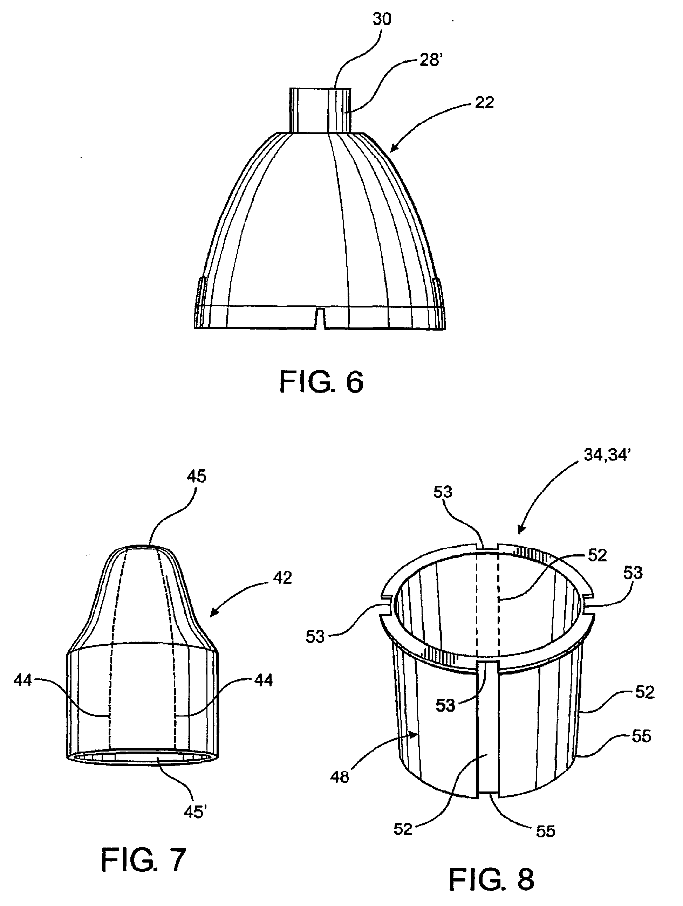

Yet additional operative components and features of the aspirator assembly 10 include a cover generally indicated as 22, which is shown in detail in Figure 6 and schematically represented in Figures 4 and 5. The cover 22 is removably connected to the casing 12 so as to be readily detached therefrom for reasons to be explained in greater detail hereinafter. As such, the cover 22 is disposed in surrounding and at least partially interconnecting relation to a base portion 24 of the casing in the manner schematically represented in Figures 4 and 5. Accordingly, the cover 22, when disposed in its intended, connected position relative to the casing 12, at least partially defines a receiving chamber 26. In addition, an inlet assembly generally indicated as 28 is formed on or otherwise connected to the cover 22 and includes an inlet opening 30 and preferably an elongated conduit 32 extending into the interior of the receiving chamber 26.

Additional features of the aspirator assembly 10, specifically relating to the receiving chamber 26 and the receipt and collection of predetermined portions of the aspirated fluid is the provision of a collection container 34. The collection container 34 is made from a disposable material and is removably placed within the receiving chamber 26, on the interior of the cover 22. More specifically, the container 34 is removably disposed in a predetermined, operative orientation which facilitates the predetermined, heavier portion of the aspirated fluid on the interior thereof. In the embodiment of Figure 4, the disposable collection container 34 is additionally structured to at least partially include a portion of a stabilizing assembly generally indicated as 36. The portion of the stabilizing assembly 36 associated with the disposable collection container 34 may include some type of indentation, recess or other appropriate structure 38. Recess 38 is dimensioned, configured and disposed to be positioned in aligned registry with a step or other protruding structure portion 40 connected to or formed as part of the casing 12. As such, the recess 38 and the step 40 are cooperatively dimensioned and configured to come into aligned registry and/or confronting engagement with one another when the container 34 is in a preferred, operative orientation. This preferred, operative orientation facilitates the collection of the heavier aspirated fluid therein by assuring that the container 34 is properly positioned relative to the inlet assembly 28 and in particular the elongated conduit 32.

Accordingly, in at least one preferred embodiment, the stabilizing assembly 36 comprises both the first portion 38 formed in or as part of the collection container 34 and the additional second portion 40 mounted on or connected to the casing 12. However, as demonstrated in Figures 5 and 8, the collection container 34' may be absent the first recessed portion 38 which at least partially defines the stabilizing assembly 36. Similarly, the second portion 40 may be completely absent from the casing 12 so as to define yet another preferred embodiment of the aspirator assembly 10 of the present invention.

Yet another structural component associated with the preferred embodiment of the aspirator assembly 10 includes a nozzle or like introductory member generally indicated as 42. The nozzle 42 is designed to be removably connected about the mouth or protruding portion 28' of the inlet assembly 28. In addition, the nozzle 42 comprises a hollow interior passage 44. The passage 44 terminates in oppositely disposed open ends 45 and 45' and as such is structured to facilitate passage of aspirated fluid from the nasal cavity in which the nozzle 42 is at least partially placed. Proper positioning of the nozzle 42 within a nasal passage is further facilitated by a substantially diverging configuration as the nozzle extends from the outer open end 45 to the inner open end 45'. The diverging configuration of nozzle 42 further facilitates its removable connection to the exterior, outwardly extending mouth 28' of the inlet assembly 28, as set forth above. It should be further noted that the nozzle 42, as well as the other cooperative components of the aspirator assembly 10 may be structured to aspirate fluid from the nasal cavity of adult individuals as well as infants and children, wherein the size of the nozzle may vary accordingly.

Intended, proper operation of the aspirator assembly 10 predicated on the establishment of fluid communication between the negative pressure source 18, the receiving chamber 26, the inlet assembly 28 and the nasal cavity of an individual. In turn communication of sufficient negative pressure with the nasal cavity necessitates the placement of the nozzle 42 at least partially therein. More specifically, the receiving chamber 26 is disposed in fluid communication with the negative pressure source 18 by virtue of a path of fluid flow extending therebetween. Accordingly, as represented in Figure 8, one preferred embodiment of the present invention includes the collection container 34 and/or 34' having an exterior surface generally indicated as 48 being structured to at least partially define the aforementioned path of fluid flow between the negative pressure source 18 and the interior of the receiving chamber 26. Such path of fluid flow at least partially extends along the exterior surface 48 and/or more specifically, the exterior wall surface 48 as schematically indicated by directional arrow 50 in Figures 4 and 5.

Therefore, the path of fluid flow 50 is even more specifically defined by one or more channels 52 formed in the exterior surface 48, wherein the channels 52 comprise elongated recesses or indented portions having oppositely disposed open ends 53 and 55. Therefore, the presence of at least one or preferably a plurality of channels 52 in the exterior surface of the collection container 34, 34' allows a gaseous portion of the aspirated fluid entering the chamber 26 to pass directly into a correspondingly disposed open end 53 of the channel 52. This directed passage of the gaseous portion of the aspirated fluid is due to the presence of negative pressure along the predetermined flow path 50 which in turn is based on fluid communication between the negative pressure source 18 and the interior of the receiving chamber 26. However, the dimension, disposition and overall structure of the one or more channels 52 and in particular open end 53 of the one or more channels 52 is such as to restrict a predetermined heavier or mucus portion of the aspirated fluid from entering into the flow path 50 and/or passing through the open end 53.

More specifically, aspirated fluid removed from the nasal cavity of an individual will comprise both mucus, representing a more dense or "heavier" portion of the aspirated fluid as well as a gaseous, lighter portion of the aspirated fluid. Accordingly, the disposition and configuration of the inlet assembly 28 specially, but not exclusively, including the elongated conduit 32 being in at least partial alignment or registry with the interior of the collection container 34, 34' results in a predetermined portion of the aspirated fluid, specifically including the heavier mucus portion, being delivered directly into the collection container 34 as schematically indicated by directional arrow 56. In contrast, the lighter or gaseous phase of the aspirated fluid will be directed, under the influence of the created negative pressure, through the open end 53 of the one or more channels 52 which at least partially define the path of fluid flow. As set forth above, the dimension and disposition of the open one or more ends 53 of the one or more channels 52 is such as to restrict passage of the heavier phase or mucus portion of the aspirated fluid.

With further reference to the embodiments of Figures 4 and 5, upon the gaseous phase of the aspirated fluid passing along the one or more channels 52, at least partially defining the predetermined path of fluid flow, it will continue to pass through appropriate seals and/or valves, as schematically represented as 60, and eventually exit through a venting assembly 62 as schematically represented by sequential directional arrows 64.

Yet another feature of at least one preferred embodiment of the present invention is represented in Figure 3. More specifically, a sound generating assembly generally indicated as 70 may also be housed on the interior of the casing 12 and be powered by the battery or other appropriate and preferably self contained power supply 20. As such, the sound generating assembly 70 may be structured to produce music or other appropriate sounds which would be especially attractive to infants and smaller children. As such, activation of the controls 14 and/or 14' would affect operation of the sound generating assembly 70 such that music or other appropriate sound could be produced concurrently to the aspirating procedure. Such sound production would have the effect of pleasing infants or small children and/or distracting them from the aspirating procedure and/or any discomfort associated therewith. Upon activation or operation, the sound generating assembly 70 will produce and/or direct sound outwardly from the casing as through appropriate apertures or openings 72 in the casing and/or in the sound generating assembly itself.

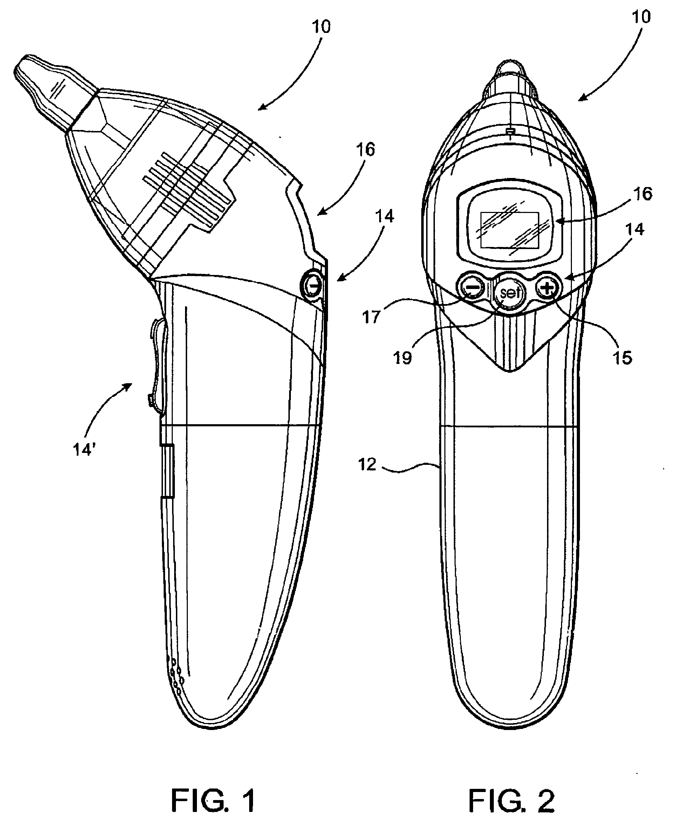

Yet another feature of the present invention is best described with primary reference to Figure 2. As represented therein, the control assembly 14 includes a plurality of operating or control buttons which may be further defined by a "plus" and "minus" buttons or control member 15 and 17 respectively. These plus and minus buttons 15 and 17 may be used in combination with a "set" button 19, to the extent that negative pressure created by the negative pressure source 18 may be increased or decreased dependent, at least in part, on the amount of suction necessary to remove the blocked mucus and/or other fluid from within the nasal cavity or passage. Once the appropriate degree of negative pressure has been determined, the set button 19 may be utilized to maintain the negative pressure at the determined value.

As should be apparent, care is taken to only use the amount of negative pressure required to remove such blockage, especially when the aspirating procedure, using the aspirating assembly 10 is performed on a small infant and/or child. As such, the controls 14 are selectively operable to effectively and accurately regulate the degree of negative pressure or suction applied to the nasal cavity or passage. In addition, the visual display 16 may be easily viewed so as to provide the operator with an indication of the degree of suction or negative pressure applied to the nasal cavity or passage of the individual being aspirated.

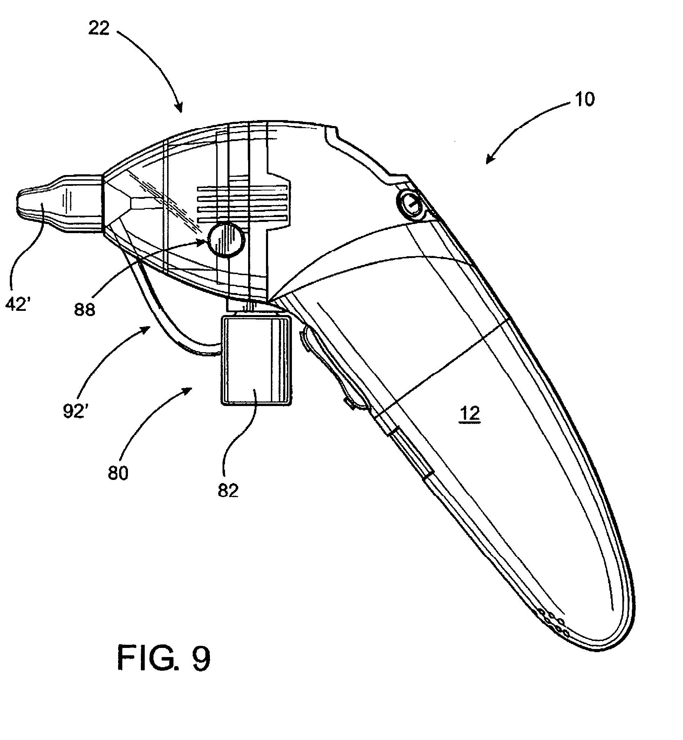

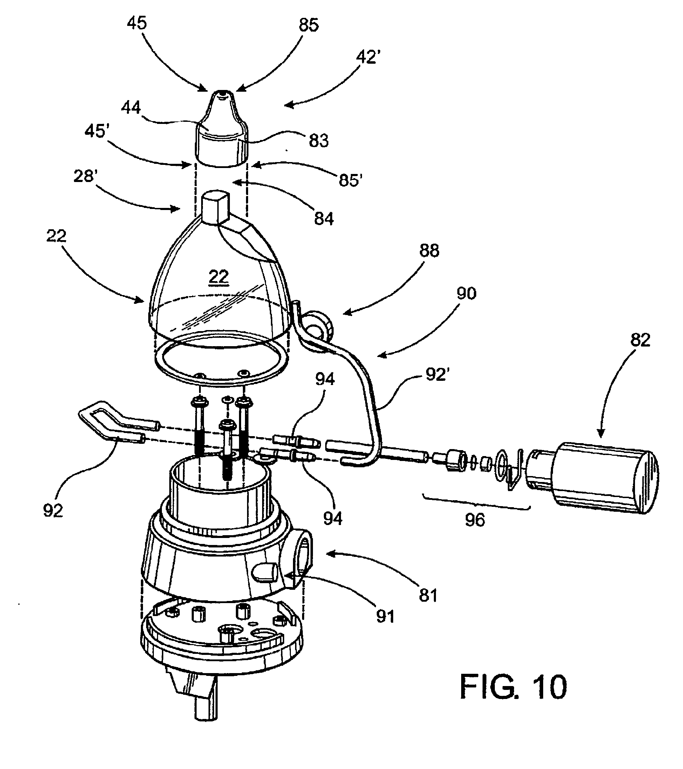

As illustrated in Figures 9 and 10, in yet another embodiment, the aspirating assembly 10 of the present invention includes a dispensing assembly, generally indicated as 80, which, as will be described in detail below, is at least partially connected to at least one or more portions of the aspirating assembly 10, such as, the casing 12, cover 22, and/or nozzle 42. In particular, the dispensing assembly 80 is operatively structured and disposed to deliver at least one liquid and/or gas to the nasal cavity or passage of an individual so as to facilitate the quick and effective removal of collected fluid from the nasal passage and/or assist with the treatment of one or more infections, colds, or mucus buildup. The liquid and/or gas deliverable to the nasal cavity via the dispensing assembly 80 may include, but is certainly not limited to water, salt water, air, oxygen, and/or any aqueous, liquid, and/or gaseous solution, and of course may include any medicinal solution or mixture structured to facilitate the practice of the present invention in the intended manner.

Moreover, the dispensing assembly 80 of the present invention comprises at least one receptacle 82 and an outlet assembly 84. The receptacle 82 is structured to hold or otherwise retain the liquid or other contents therein, at least until the liquid is expelled or delivered therefrom, for example, to the outlet assembly 84 and/or nasal cavity, as will be discussed below. In particular, the receptacle 82 and outlet assembly 84 are disposed in a fluid communication with one another, for instance via at least one connector assembly 90. Specifically, the connector assembly 90 comprises at least one or a plurality of tubes 92 and/or connectors 94 each comprising an at least partially hollow interior configuration so as to allow the liquid to flow therethrough. As such, in at least one embodiment of the present invention, the connector assembly 90 is coupled to the receptacle 82 and the outlet assembly 84. Of course the connector assembly 90 may, in at least one embodiment, comprise additional connecting components 96 such as one or more gaskets, washers, pins, or other holding, connecting, and/or stabilizing mechanisms.

In addition, at least one embodiment comprises a plurality of interchangeable receptacles 82 and/or a removable, refillable, and/or reusable receptacle 82. As such, the various additional connecting components 96 of at least one embodiment are structured to facilitate the removable interconnection of the one or more receptacles 82 to the casing 12, cover 22, or other portions of the aspirator assembly 10 for instance, via a receptacle port 81. In particular, the receptacle(s) 82 may be removably or fixedly connected to the aspirator assembly 10 in any one or more manners, including, for example, via cooperatively structured threading, grooves, notches, snaps, latches, etc.

Additionally, in at least one embodiment, the receptacle 82 and/or at least a portion 92' of the tube(s) 92 is disposed in an external relation relative to the casing 12 and/or cover 22. In particular, the tube 92, 92' and/or any other portion of the connector assembly 90 may be structured to pass through a connecting port 91 and disposed on or proximate an external surface of the cover 22 and, for example, at least partially disposed within a cooperatively structured groove 89 disposed on or otherwise defined by the external peripheral surface 22' of the cover 22. The connector assembly 90 of at least one embodiment is further structured to connect or couple with the outlet assembly 84 at or near the nozzle 42'. Accordingly, the dispensing assembly 80 and/or the connector assembly 90 thereof of at least one embodiment is operatively structured and disposed so as to be isolated from and not interfere with the receiving chamber 26 and/or collection container 34. Thus, at least one embodiment of the present invention is structured and disposed to minimize and/or eliminate any possible obstruction from the dispensing assembly 80 or the connector assembly 90 thereof with the collection of aspirated fluid as discussed in detail above.

Furthermore, the outlet assembly 84 of the present invention is connectable or coupled to the nozzle 42', and, in at least one embodiment, comprises an outlet opening (not illustrated) which is structured and disposed to deliver the liquid to the nasal cavity. Specifically, as illustrated in Figure 10, the outlet assembly 84 of at least one embodiment of the present invention is operatively disposed in a substantially adjacent relation to the inlet assembly 28 and/or the protruding portion 28' thereof. In at least one embodiment, the outlet assembly 84 is thus disposed on an external peripheral surface 22' of the cover 22 and is thereby isolated or separated from the receiving chamber 20 and/or the aspirated fluid contained therein. Moreover, the nozzle 42' is structured and configured to be disposable in a substantially surrounding and simultaneously connecting relation to the protruding portion 28' of the inlet assembly 28 and the outlet assembly 84.

More in particular, the nozzle 42' of at least one embodiment comprises at least two passages 44 and 83. As discussed in detail above, the passage 44 terminates in oppositely disposed open ends 45 and 45' and as such is structured to facilitate passage of aspirated fluid from the nasal cavity in which the nozzle 42' is at least partially placed. Similarly, passage 83 terminates in oppositely disposed open ends 85 and 85' and is structured to facilitate passage of liquid from the dispensing assembly 80 to the nasal cavity. Of course, the liquid may be delivered from the receptacle to the nasal cavity via passage 83, and subsequently pass back through the nozzle 42' via passage 44 and through the inlet assembly 28 in a similar fashion as the aspirated fluid as discussed in greater detail above.

Furthermore, the dispensing assembly 80 of at least one embodiment of the present invention is structured to selectively deliver the liquid from the receptacle to the outlet assembly 84 and into the nasal cavity. In particular, at least one embodiment includes an actuator assembly 88 structured to at least partially control a flow of liquid to the nasal cavity, for instance upon manipulation or pressing of the actuator assembly 88. As such, in at least one embodiment, the actuator assembly 88 is connected or coupled to the connector assembly 90 and accessible from a position external to the casing 12. Thus, an individual may manipulate or press the actuator assembly 88, which is structured to selectively release liquid or otherwise allow liquid to flow through the connector assembly 90, to the outlet assembly 84, and into the nasal cavity.

Moreover, in at least one embodiment, the receptacle 82, and/or the contents contained therein, is pre-pressurized such that the actuator assembly 88, when manipulated, is structured to release at least some of the pressure contained therein, and thus allow the liquid disposed within the receptacle 82 to flow therefrom and into the nasal cavity, as discussed above. Accordingly, in at least one embodiment, the actuator assembly 88 comprises a movable obstruction, blocking device, and/or valve relative to the at least partially hollow interior of one or more components of the connector assembly 90, the valve being disposable between an obstructing orientation and an open orientation relative to the connector assembly 90, receptacle 82, outlet assembly 84 or any other portion of the dispensing assembly 80 of the present invention.

In yet another embodiment, the receptacle 82 and/or connector assembly 90 is connected or coupled to the negative pressure source 18 or other like mechanism which is cooperatively structured and disposed to pressurize the receptacle 82 and/or the contents therein. As such, in at least one embodiment, upon connecting the receptacle 82 to the aspirator assembly 10, the negative pressure source 18 or other structure may selectively or automatically pressurize the receptacle 82 and/or the connector assembly 90 such that, upon manipulation or pressing of the actuator assembly 88, liquid contained within the receptacle 82 may flow to the nasal cavity.

In addition, the negative pressure source 18 or other like mechanism of at least one embodiment may be structured to create a vacuum within the connector assembly 90 so as to fill the connector assembly 90 with the liquid contained within the receptacle 82 or otherwise create a flow of liquid in the connector assembly 90, at least to the point of a valve or other obstruction device. Particularly, the negative pressure source 18 may function in a manner so as to create negative pressure within the connector assembly 90 and/or function in a manner similar to a siphon assembly. Thus, manipulation and/or pressing of the actuator assembly 88 is structured to open the valve disposed along the connector assembly 90 so as to allow the flow of liquid to pass therethrough and into the nasal passage.

In another embodiment, manipulation and/or successive manipulation of the actuator assembly 88 is structured to at least temporarily create a vacuum within the connector assembly 90. As such, the first few pumps or manipulations of the actuator assembly 88 may but need not expel air from the connector assembly 90 and thereby prime the connector assembly 90 and/or cause the connector assembly 90 to fill with liquid from the receptacle 82. Successive pumping or manipulation of the actuator assembly 88 will thus deliver the liquid to the nasal cavity and thus function in a manner somewhat akin to a pump-activated spray bottle.

In a further embodiment of the present invention, upon manipulation of the actuator assembly 88, the aspirator assembly 10 of the present invention is structured to be selectively disposed into and out of an aspirating mode and a dispensing mode. In particular, the aspirating mode is defined wherein the aspirator assembly 10 is configured and positioned to aspirate fluid, for example, from the nasal cavity of an individual as explained in detail herein. The dispensing mode is defined wherein the aspirator assembly 10 of the present invention is configured and positioned to dispense liquid and/or gas, for example, into the nasal cavity of an individual via the dispensing assembly 80. Accordingly, upon selective manipulation or pressing of the actuator assembly 88, at least one embodiment of the present invention is structured to cease aspirating and begin to dispense liquid into the nasal cavity. The aspirating assembly 10 may dispense a predetermined amount of liquid and/or gas upon the manipulation of the actuator assembly 88 and automatically or selectively return to the aspirating mode. However, in at least one embodiment, the aspirator assembly 10 is structured to continuously dispense liquid and/or gas until the liquid and/or gas is used up or until the actuator assembly 88 is released. At that time, the aspirator assembly 10 may automatically and/or selectively return to the aspirating mode, as described above.

More in particular, the negative pressure source 18 of the present invention may, in at least one embodiment, be structured to stop aspirating upon the manipulation of the actuator assembly 88 and begin to direct or pump air or other substance into the receptacle 82, for instance via an assembly of one or more connecting tubes, so as to cause the liquid and/or gas contained therein to be delivered to the outlet assembly 84 and/or nasal cavity. In particular, the liquid and/or gas contained within the receptacle 82 may be forced out of the receptacle 82 and into the outlet assembly upon the presence of forced air or other substance within the receptacle 82. It should be apparent however that, the present invention may further comprise a pump or other positive pressure source which is structured to pump or direct air or other substance into the receptacle 82 or dispensing assembly 80. Accordingly, upon manipulation of the actuator assembly 88, the pump of at least one embodiment may be structured to direct air or other substance into the receptacle 82 so as to cause the liquid and/or gas contained therein to be directed to the nasal cavity, as discussed above.

Since many modifications, variations and changes in detail can be made to the described preferred embodiment of the invention, it is intended that all matters in the foregoing description and shown in the accompanying drawings be interpreted as illustrative and not in a limiting sense. Thus, the scope of the invention should be determined by the appended claims and their legal equivalents.

## Claims

1. An assembly structured (10) to aspirate fluid from a nasal cavity, said assembly (10) comprising: a casing (12) including an at least partially hollow interior, a cover (22) structured to at least partially define a receiving chamber (26) on an interior thereof when said cover (22) is connected to said casing (12), a negative pressure source (18) disposed within said casing and in fluid communication with said receiving chamber (26), and an inlet assembly (28) disposed in communicating relation with said receiving chamber (26) and structured to direct aspirated fluid into said receiving chamber, the assembly (10) comprising a collection container (34, 34') removably disposed within said receiving chamber (26) and disposed to collect the aspirated fluid therein, **characterized in that** said collection container (34, 34') comprises at least one or a plurality of channels (52) formed in its exterior surface allowing a gaseous portion of the aspirated fluid entering said receiving chamber (26) to be directed out from the interior of said receiving chamber (26) through the channels (52) while restricting the passage of a heavier portion or mucus portion of the aspirated fluid.

2. An assembly (10) as recited in claim 1, **characterized in that** the assembly also comprises seals and /or valves and a venting assembly (62), so that the gaseous portion of the aspirated fluid after passing through the channels (52), continue to pass through the seals and/or the valves and exit through the venting assembly.

3. An assembly (10) according to any claims 1 to 2, wherein said channels (52) comprising elongated recesses having oppositely open ends (53, 55).

4. An assembly (10) as recited in claim 3, wherein at least a first of said open ends (53) is disposed in communicating relation with the aspirated fluid received within said receiving chamber (26), said first open end (53) further disposed and structured to restrict passage of the heavier portion of the aspirated fluid from passing threrethough.

5. An assembly (10) according to any claims 1 to 4, characterized it comprises a dispensing assembly (80) at least partially connected to said casing (12) and being structured to deliver at least one liquid to the nasal cavity.

6. An assembly (10) as recited in claim 5, wherein said dispensing assembly (80) comprises a receptacle (82) and an outlet assembly (84), said receptacle (82) being disposed in a fluid communication with said outlet assembly (84).

7. An assembly (10) as recited in claim 6, wherein said receptacle (82) is also removable in order to be substituted with another one.

8. An assembly (10) as recited in claim 7, wherein said receptacle (82) and said outlet assembly (84) are disposed in said fluid communication with one another via at least one connector assembly (90).

9. An assembly (10) as recited in claim 6, further comprising a nozzle (42') connected to said outlet assembly (84), said nozzle (42') being structured and disposed to direct said at least one liquid from said outlet assembly (84) to the nasal cavity.

10. An assembly (10) as recited in claim 6, wherein said outlet assembly (84) is operatively disposed in a substantially adjacent relation to said inlet assembly (28).

11. An assembly (10) as recited in claim 9, wherein said nozzle (42') is disposable in substantially surrounding relation to at least a portion of said inlet assembly (28) and said outlet assembly (84).

12. An assembly (10) as recited in claim 11, wherein said nozzle (42') is dimensioned and configured to enter into the nasal cavity.

13. An assembly (10) as recited in claim 12, wherein said dispensing assembly (80) is structured to selectively deliver said liquid to the nasal cavity.

14. As assembly (10) as recited in claim 13, wherein said dispensing assembly (80) further comprises an actuator assembly (88) structured to at least partially control a flow of said liquid to the nasal cavity.

15. An assembly (10) as recited in claim 14, wherein said actuator assembly (88) is at least partially coupled to said connector assembly (90).

## Patentansprüche

1. Anordnung, die strukturiert (10) ist, um Fluid von einer Nasenhöhle abzusaugen, wobei die Anordnung (10) Folgendes umfasst:
ein Gehäuse (12), umfassend ein mindestens teilweise hohles Inneres, eine Abdeckung (22), die strukturiert ist, um mindestens teilweise eine Aufnahmekammer (26) auf einem Inneren davon zu definieren, wenn die Abdeckung (22) mit dem Gehäuse (12) verbunden ist, eine Unterdruckquelle (18), die in dem Gehäuse angeordnet und in fluidischer Kommunikation mit der Aufnahmekammer (26) ist, und eine Einlassanordnung (28), die in kommunizierender Beziehung mit der Aufnahmekammer (26) angeordnet und strukturiert ist, um das abgesaugte Fluid in die Aufnahmekammer zu richten, wobei die Anordnung (10) einen Sammelbehälter (34, 34') umfasst, der entfernbar in der Aufnahmekammer (26) angeordnet ist und angeordnet ist, um das abgesaugte Fluid darin zu sammeln, **dadurch gekennzeichnet, dass** der Sammelbehälter (34, 34') mindestens einen oder eine Vielzahl von Kanälen (52) umfasst, gebildet in der Außenfläche, der/die ermöglicht, dass ein gasförmiger Teil des abgesaugten Fluids in die Aufnahmekammer (26) eintritt, um durch die Kanäle (52) aus dem Inneren der Aufnahmekammer (26) gerichtet zu werden, während der Durchgang eines schwereren Teils oder eines schleimigen Teils des abgesaugten Fluids beschränkt wird.

2. Anordnung (10) nach Anspruch 1, **dadurch gekennzeichnet, dass** die Anordnung auch Dichtungen und/oder Ventile und eine Entlüftungsanordnung (62) umfasst, so dass der gasförmige Teil des abgesaugten Fluids, nachdem er durch die Kanäle (52) passiert ist, weiterhin durch die Dichtungen und/oder die Ventile passiert, und durch die Belüftungsanordnung austritt.

3. Anordnung (10) nach einem der Ansprüche 1 bis 2, wobei die Kanäle (52) verlängerte Vertiefungen mit einander gegenüber liegenden offenen Enden (53, 55) umfassen.

4. Anordnung (10) nach Anspruch 3, wobei mindestens ein erstes der offenen Enden (53) in kommunizierender Beziehung mit dem abgesaugten Fluid angeordnet ist, das in der Aufnahmekammer (26) aufgenommen wird, wobei das erste offene Ende (53) weiter angeordnet und strukturiert ist, um den Durchgang des schwereren Teils des abgesaugten Fluids dadurch zu beschränken.

5. Anordnung (10) nach einem der Ansprüche 1 bis 4, **dadurch gekennzeichnet, dass** sie eine Abgabeanordnung (80) umfasst, die mindestens teilweise mit dem Gehäuse (12) verbunden und strukturiert ist, um mindestens ein Fluid an die Nasenhöhle abzugeben.

6. Anordnung (10) nach Anspruch 5, wobei die Abgabeanordnung (80) ein Aufnahmegefäß (82) und eine Auslassanordnung (84) umfasst, wobei das Aufnahmegefäß (82) in einer fluidischen Kommunikation mit der Auslassanordnung (84) angeordnet ist.

7. Anordnung (10) nach Anspruch 6, wobei das Aufnahmegefäß (82) ebenfalls entfernbar ist, um durch ein anderes ausgetauscht zu werden.

8. Anordnung (10) nach Anspruch 7, wobei das Aufnahmegefäß (82) und die Auslassanordnung (84) über mindestens eine Verbindungsanordnung (90) in der fluidischen Kommunikation miteinander angeordnet sind.

9. Anordnung (10) nach Anspruch 6, weiter umfassend eine Düse (42'), die mit der Auslassanordnung (84) verbunden ist, wobei die Düse (42') strukturiert und angeordnet ist, um das mindestens eine Fluid von der Auslassanordnung (84) an die Nasenhöhle zu richten.

10. Anordnung (10) nach Anspruch 6, wobei die Auslassanordnung (84) im Betrieb in einer im Wesentlichen benachbarten Beziehung zu der Einlassanordnung (28) angeordnet ist.

11. Anordnung (10) nach Anspruch 9, wobei die Düse (42') in einer im Wesentlichen umgebenden Beziehung zu mindestens einem Abschnitt der Einlassanordnung (28) und der Auslassanordnung (84) angeordnet werden kann.

12. Anordnung (10) nach Anspruch 11, wobei die Düse (42') abgemessen und konfiguriert ist, um in die Nasenhöhle einzutreten.

13. Anordnung (10) nach Anspruch 12, wobei die Abgabeanordnung (80) strukturiert ist, um selektiv das Fluid an die Nasenhöhle abzugeben.

14. Anordnung (10) nach Anspruch 13, wobei die Abgabeanordnung (80) weiter eine Aktuatoranordnung (88) umfasst, die strukturiert ist, um mindestens teilweise einen Fluss des Fluids an die Nasenhöhle zu steuern.

15. Anordnung (10) nach Anspruch 14, wobei die Aktuatoranordnung (88) mindestens teilweise mit der Verbindungsanordnung (90) gekoppelt ist.

## Revendications

1. Ensemble (10) structuré pour aspirer un fluide d'une cavité nasale, ledit ensemble (10) comprenant : un boîtier (12) comprenant une partie intérieure au moins partiellement creuse, un élément de recouvrement (22) structuré pour au moins définir partiellement une chambre de réception (26) sur une partie intérieure de celui-ci lorsque ledit élément de recouvrement (22) est relié au dit boîtier (12), une source de pression négative (18) disposée dans ledit boîtier et en communication fluidique avec ladite chambre de réception (26), et un ensemble d'entrée (28) disposé dans une relation de communication avec ladite chambre de réception (26) et structuré pour diriger le fluide aspiré dans ladite chambre de réception, l'ensemble (10) comprenant un contenant de collecte (34, 34') disposé de manière amovible dans ladite chambre de réception (26) et disposé pour collecter le fluide aspiré dans celui-ci, **caractérisé en ce que** ledit contenant de collecte (34, 34') comprend au moins un canal ou une pluralité de canaux (52) formés dans sa surface extérieure, permettant qu'une partie gazeuse du fluide aspiré entrant dans ladite chambre de réception (26) soit dirigée hors de la partie intérieure de ladite chambre de réception (26) à travers les canaux (52) tout en limitant le passage d'une partie plus lourde ou d'une partie de mucus du fluide aspiré.

2. Ensemble (10) selon la revendication 1, **caractérisé en ce que** l'ensemble comprend également des joints et/ou valves et un ensemble formant évent (62), de sorte que la partie gazeuse du fluide aspiré après être passée à travers les canaux (52) continue de passer à travers les joints et/ou les valves et sorte à travers l'ensemble formant évent.

3. Ensemble (10) selon l'une quelconque des revendications 1 et 2, dans lequel lesdits canaux (52) comprennent des évidements allongés ayant des extrémités ouvertes (53, 55) opposées.

4. Ensemble (10) selon la revendication 3, dans lequel au moins une première desdites extrémités ouvertes (53) est disposée dans une relation de communication avec le fluide aspiré reçu dans ladite chambre de réception (26), ladite première extrémité ouverte (53) étant en outre disposée et structurée pour limiter le passage de la partie plus lourde du fluide aspiré à travers celle-ci.

5. Ensemble (10) selon l'une quelconque des revendications 1 à 4, **caractérisé en ce qu'**il comprend un ensemble de distribution (80) relié au moins partiellement au dit boîtier (12) et structuré pour délivrer au moins un liquide dans la cavité nasale.

6. Ensemble (10) selon la revendication 5, dans lequel ledit ensemble de distribution (80) comprend un réceptacle (82) et un ensemble de sortie (84), ledit réceptacle (82) étant disposé en communication fluidique avec ledit ensemble de sortie (84).

7. Ensemble (10) selon la revendication 6, dans lequel ledit réceptacle (82) peut également être retiré afin d'être remplacé par un autre.

8. Ensemble (10) selon la revendication 7, dans lequel ledit réceptacle (82) et ledit ensemble de sortie (84) sont disposés en ladite communication fluidique l'un avec l'autre par l'intermédiaire d'au moins un ensemble formant raccord (90).

9. Ensemble (10) selon la revendication 6, comprenant en outre une buse (42') reliée au dit ensemble de sortie (84), ladite buse (42') étant structurée et disposée pour diriger ledit au moins un liquide dudit ensemble de sortie (84) vers la cavité nasale.

10. Ensemble (10) selon la revendication 6, dans lequel ledit ensemble de sortie (84) est disposé de manière fonctionnelle dans une relation sensiblement adjacente au dit ensemble d'entrée (28).

11. Ensemble (10) selon la revendication 9, dans lequel ladite buse (42') peut être disposée dans une relation sensiblement d'encerclement par rapport à au moins une partie dudit ensemble d'entrée (28) et dudit ensemble de sortie (84).

12. Ensemble (10) selon la revendication 11, dans lequel ladite buse (42') est dimensionnée et configurée pour pénétrer dans la cavité nasale.

13. Ensemble (10) selon la revendication 12, dans lequel ledit ensemble de distribution (80) est structuré pour délivrer de manière sélective ledit liquide à la cavité nasale.

14. Ensemble (10) selon la revendication 13, dans lequel ledit ensemble de distribution (80) comprend en outre un ensemble formant actionneur (88) structuré pour commander au moins partiellement un écoulement dudit liquide vers la cavité nasale.

15. Ensemble (10) selon la revendication 14, dans lequel ledit ensemble formant actionneur (88) est au moins partiellement couplé au dit ensemble formant raccord (90).
